# DEMANDE DE BREVET EUROPEEN

(11) **EP 4 442 244 A1**
(43) Date de publication de la demande: **09.10.2024**
(21) Numéro de dépôt: 24162611.8
(22) Date de dépôt: 11.03.2024
(51) Int. Cl.: A61K 8/37, A61K 8/87, A61Q 17/04, A61K 8/85

(54) **COMPOSITION COSMETIQUE COMPRENANT UN FILTRE UVA ET/OU UVB, UN AGENT FILMOGENE LIPOPHILE ET UN AGENT FILMOGENE HYDROPHILE**

(30) Priorité: 14.03.2023 FR 2302352
(71) Demandeur: Laboratoires De Biologie Vegetale Yves Rocher, 56200 La Gacilly (FR)
(72) Inventeur: JOLITON, Hugo, 75004 PARIS (FR); DIEU, Jessica, 78000 VERSAILLES (FR); DE LONGVILLIERS, Clémence, 75004 PARIS (FR)
(74) Mandataire: Novagraaf Technologies

(57) **Abrégé**

[1] L'invention appartient au domaine de la cosmétique, notamment la protection des matières kératiniques (peau et/ou cheveux), de préférence des lèvres et/ou de la peau (visage et/ou corps), plus particulièrement à celui des compositions telles que des compositions de protection solaire.
[2] La présente invention se rapporte à une composition cosmétique comprenant au moins un filtre UVA et/ou UVB, un agent filmogène lipophile comprenant du diisostearoyl polyglyceryl 3 dimer dilinoleate et un agent filmogène hydrophile comprenant un polyuréthane. La composition selon l'invention est une composition cosmétique de protection solaire, et notamment une composition anti-UVA et/ou anti-UVB. La composition selon l'invention se présente sous la forme d'une émulsion eau-dans-huile ou huile-dans-eau. L'invention se rapporte aussi à l'utilisation d'une combinaison d'un agent filmogène lipophile comprenant du diisostearoyl polyglyceryl 3 dimer dilinoleate et d'un agent filmogène hydrophile comprenant un polyuréthane dans une composition cosmétique de protection solaire.

## Description

L'invention appartient au domaine de la cosmétique, notamment la protection des matières kératiniques (peau et/ou cheveux), de préférence des lèvres et/ou de la peau (visage et/ou corps), plus particulièrement à celui des compositions telles que des compositions de protection solaire.

La présente invention se rapporte à une composition cosmétique comprenant au moins un filtre UVA et/ou UVB, un agent filmogène lipophile comprenant du diisostearoyl polyglyceryl 3 dimer dilinoleate et un agent filmogène hydrophile comprenant un polyuréthane. La composition selon l'invention est une composition cosmétique de protection solaire, et notamment une composition anti-UVA et/ou anti-UVB. La composition selon l'invention se présente sous la forme d'une émulsion eau-dans-huile ou huile-dans-eau. L'invention se rapporte aussi à l'utilisation d'une combinaison d'un agent filmogène lipophile comprenant du diisostearoyl polyglyceryl 3 dimer dilinoleate et d'un agent filmogène hydrophile comprenant un polyuréthane dans une composition cosmétique de protection solaire.

La demanderesse est créatrice de la Cosmétique Végétale et promeut une cosmétique naturelle depuis 1959. Sa recherche de matières premières et son expertise en formulation la pousse à innover pour créer des formules toujours plus naturelles pour satisfaire le consommateur et créer de la valeur scientifique. Afin de limiter son impact sur l'environnement, la demanderesse mène une démarche d'innovation pour substituer les ingrédients issus de ressources non renouvelables et non facilement biodégradables. Ceci représente un challenge majeur dans certaines catégories cosmétiques, car il est impératif de garantir en parallèle la sécurité, l'efficacité et la sensorialité des produits auprès des consommateurs.

### Etat de la technique

Les compositions de protection solaire, de préférence anti-UVA et/ou anti-UVB, sont connues depuis de nombreuses années.

Les effets du soleil dépendent avant tout du phototype du sujet exposé qui correspond à son l'aptitude à bronzer. Plus le phototype est faible, plus le sujet sera sensible aux effets du soleil. L'exposition au soleil engendre des effets visibles à court terme. Le plus connu est l'érythème actinique, vulgairement connu sous le nom de coup de soleil. Ce phénomène a lieu principalement sous l'action des UVB et dans une moindre mesure à cause des UVA. Les effets des UVB se caractérisent par une réponse sous la forme d'érythème dont l'intensité maximale se situe entre 6 et 24 heures après exposition. L'érythème induit par les UVA se manifeste lui encore 48 à 72 heures après exposition. Une pigmentation immédiate de la peau est constatée dans les minutes qui suivent le début de l'exposition et s'atténue dans les heures qui suivent. Cette réaction provoquée par les UVA est suivie d'une pigmentation persistante de la peau 2 à 24 heures après exposition. Ces deux phénomènes sont provoqués par une photo-oxydation des mélanines présentes dans l'épiderme sans qu'elles soient renouvelées. Le bronzage, auquel on adjoint le terme retardé n'apparait que 48 à 72 heures après l'exposition, se maintient pendant plusieurs semaines en s'atténuant lentement. Il est induit par les UVA et les UVB, la réponse de la peau est une augmentation de l'activité de la tyrosinase qui renouvelle les mélanines. Ces réactions immédiates sont les plus connues par le grand public. Des réactions de photo-immunosuppression peuvent également être constatées après exposition aux UV. Ce phénomène se manifeste notamment par une réapparition de l'herpès **(1,2)**.

L'exposition au soleil comporte également des effets chroniques. Un vieillissement actinique cutané est constaté, celui-ci est majoritairement provoqué par les UVA. Ils pénètrent plus profondément dans l'épiderme et produisent des dérivés réactifs de l'oxygène (en anglais ROS pour *reactive oxygen species*) tels que des radicaux libres et des peroxydes. Ces ROS vont endommager l'ADN, les protéines et les membranes cellulaires, et altérer l'élastine et le collagène de la matrice extracellulaire. Des manifestations cliniques sont des lentigos actiniques, des comédons ouverts de taille importante, une perte d'élasticité de la peau et l'apparition de rides. Mais le risque le plus redouté est la photo-carcinogenèse. La causalité entre une intense exposition aux rayons UV et le développement des trois types de cancers cutanés principaux qui sont le mélanome, le carcinome basocellulaire et le carcinome spinocellulaire a été mise en évidence. Les UVB peuvent provoquer la nécrose des cellules de l'épiderme entrainant un désordre moléculaire précurseur de cancers cutanés. Les UVA par leur production d'espèces radicalaires permettent aussi l'émergence de cellules cancéreuses. Il est aujourd'hui établi que les coups de soleil qui se sont déclarés durant l'enfance constituent une cause des cancers cutanés à l'âge adulte **(1,3).**

Pour ces raisons, il est important de se protéger tant des UVA que des UVB et les crèmes solaires sont une protection efficace contre ces rayonnements nocifs.

Mais l'utilisation massive de crème solaire, en particulier dans les régions touristiques présente un certain nombre d'inconvénients, en particulier pour l'environnement.

Il est ainsi estimé que chaque année, entre 6 000 et 14 000 tonnes de crèmes solaires (soit environ 50 000 flacons) sont diluées dans les eaux des zones de récifs coralliens et 80 millions de touristes se rendent dans des zones de baignade corallienne. Une des conséquences d'une telle activité est la participation au blanchiment des coraux qui est plus que jamais d'actualité avec la récente législation qu'a appliquée Hawaï à ce sujet **(4,5).**

Dans le monde, sur une surface totale de récifs et de lagons de 600 000 km² (280 000 km² de récifs), on estime que 20% des récifs coralliens ont été irrémédiablement détruits ou présentent peu de chances de récupération, que 25% sont dans un état critique, que 25% sont menacés et que seulement 30% demeurent dans un état satisfaisant. Parmi les nombreuses pathologies touchant les coraux, le blanchiment corallien est celle qui augmente le plus en fréquence et en sévérité. Lorsque l'environnement physicochimique du corail varie : augmentation de la température de l'eau de mer, de l'acidité ou des radiations UV, présence de bactéries pathogènes ; il a été observé que la réponse la plus commune à ce genre de stress est l'expulsion des zooxanthelles. Les coraux qui tirent leurs couleurs des zooxanthelles qui les couvrent deviennent alors blancs. Ce phénomène de blanchiment et réversible dès lors que les conditions environnementales du corail deviennent moins stressantes, les zooxanthelles reprennent alors leur place. En revanche puisqu'ils apportent également 90% des ressources énergétiques aux coraux, si l'effet de stress et donc l'expulsion des coraux sont prolongés les coraux finissent par mourir. On observe après ces phénomènes la déplétion d'autres espèces marine formant la biodiversité environnant les coraux. En sus de cet impact écologique les revenues liés à l'industrie touristique diminuent également, cette perte est estimée à 90 milliards de dollars par an pour le tourisme caribéen **(6).**

Des études ont été menées afin de rechercher l'impact que les filtres UV pouvaient avoir sur l'environnement marin. Il ressort de ces études que les effets de l'oxybenzone (benzophénone-3) ont été particulièrement étudiés. Il a été observé qu'une augmentation de la concentration en oxybenzone était associée au blanchiment corallien. L'effet de produits de protection solaire a été étudié in situ sur des coraux dans les Océans Atlantique, Pacifique, Indien et en mer Rouge. Sur ces mêmes coraux ont également été testés six filtres solaires organiques largement retrouvé dans les préparations solaires : butylmethoxydibenzoylmethane (avobenzone), 4-methylbenzylidene camphor, ethylhexylsalicylate, benzophenone-3 (oxybenzone), octocrylene et ethylhexylmethoxycinnamate (octinoxate). Là encore des phénomènes de blanchiment ont pu leur être imputés **(7,8)**.

Les filtres UV organiques ne sont pas les seuls responsables, et il a également été démontré que les filtres inorganiques ne sont pas non plus inoffensifs. Le TiO2 et le ZnO ont été étudiés sous leur forme nano et leur toxicité sur le développement de phytoplancton est avéré **(9).**

Des autorités politiques se sont également emparées du sujet. Le gouvernement de l'état de Hawaï a été le premier, en juillet 2018, à légiférer l'interdiction de deux filtres solaires, la benzophénone-3 et l'ethylhexylmethoxycynnamate, qui prendra effet au 1er janvier 2021. En France, au mois d'août 2018, il a été annoncé le secrétaire d'Etat auprès du ministre de la Transition Ecologique et Solidaire, le lancement d'une « Mission Océan ». Au cours de celle-ci, l'ANSES (Agence Nationale de la Sécurité Sanitaire de l'Alimentation, de l'Environnement et du Travail) sera chargée de réaliser « un inventaire des différentes substances chimiques présentes dans les produits de consommation comme les cosmétiques, les crèmes solaires ou les lessives et particulièrement toxiques pour les écosystèmes marins et les récifs coralliens » **(10,11).**

Malgré les multiples revendications des fabricants de respect de l'environnement marin, il reste compliqué de trouver une composition qui minimise son impact sur celui-ci.

Il y a donc un réel besoin de mettre à disposition une composition solaire qui puisse limiter ou éviter les phénomènes décrits ci-avant, c'est-à-dire une composition permettant une forte protection face au rayons UV et une forte résistance à l'eau qui témoigne d'une dilution moindre, voire nulle, dans les eaux de baignade.

Classiquement, on entend par composition de protection solaire, un produit cosmétique, le plus souvent sous forme d'émulsion, destiné à protéger l'utilisateur contre les rayonnements UVA et/ou UVB par l'ajout de filtres UV organiques et/ou inorganiques, ceux-ci étant dans le cadre de l'Union Européenne listés à l'annexe VI du règlement (CE) 1223/2009. Les compositions de protection solaire classiques possèdent de nombreux avantages tels que notamment une protection plus ou moins élevée en fonction des besoins d'utilisation. Elles possèdent cependant plus rarement une résistance à l'eau qui permet le maintien d'une protection solaire dans des conditions ou les matières kératiniques à protéger sont susceptibles d'être mouillées et/ou immergées, sans baisse significative de l'efficacité de la composition mais également sans déversement dans l'environnement de produits chimiques polluants (filtres UV). Ainsi malgré des avantages certains, les compositions cosmétiques de protection solaire classiques conservent à ce jour certaines contraintes et sont polluantes. Il subsiste ainsi une difficulté d'obtenir à la fois une bonne protection contre les UVA et/ou les UVB tout en ayant une résistance suffisante à l'eau.

Il existe ainsi un besoin de proposer de nouvelles compositions cosmétiques de protection solaire qui résolvent les problèmes observés dans les compositions connues. Notamment, il existe un besoin de proposer de nouvelles compositions cosmétiques de protection solaire qui permettent de conférer une capacité de protection adaptée et optimisée, tout en ayant une résistance accrue à l'eau.

### Description détaillée de l'invention

Il est du mérite de la demanderesse de proposer une nouvelle composition comprenant au moins un filtre UVA et/ou UVB, un agent filmogène lipophile comprenant du diisostearoyl polyglyceryl 3 dimer dilinoleate et un agent filmogène hydrophile comprenant un polyuréthane et qui, lorsqu'elles sont incorporées dans une composition cosmétique lui confère une propriété de protection contre les UVA et/ou les UVB adaptée et optimisée, notamment grâce à une résistance à l'eau bien supérieure à celle d'une composition ne comprenant pas la combinaison d'agents filmogènes selon l'invention, ou comprenant une alternative issus de ressources non renouvelables et non facilement biodégradables reconnue par une personne du métier. La nouvelle composition selon l'invention permet une durée de protection fortement prolongée par rapport au compositions classiques. La composition selon l'invention peut procurer une protection solaire améliorée allant jusqu'à augmenter le SPF de plus de dix points par rapport à une composition comprenant une alternative synthétique reconnu par une personne du métier. La composition selon l'invention permet d'obtenir un produit de protection solaire résistant à l'eau et même très résistant à l'eau selon les normes d'évaluations en vigueurs, par rapport à une composition comprenant une alternative synthétique reconnue par une personne du métier. La combinaison d'agents filmogènes selon l'invention permet ainsi d'obtenir un effet synergique entre les propriétés de protection solaire et de résistance à l'eau de la composition, et permet ainsi d'adapter et d'optimiser la protection solaire en fonction de l'utilisation. La plus grande efficacité de protection tant en indice SPF qu'en durée, permet en outre de limiter la quantité de filtres UVA et/ou UVB à appliquer ou de limiter le besoin d'en réappliquer sur la peau après une baignade.

La composition cosmétique selon l'invention est très versatile et peut notamment être incorporé à tout type d'émulsions eau-dans-huile ou huile-dans-eau, telle qu'une crème, un lait, un spray, une émulsion coulée et/ou d'une émulsion colorée telle qu'une BB crème, un fond de teint.

L'invention se rapporte ainsi à une composition cosmétique comprenant :
- au moins un filtre UVA et/ou UVB,
- un agent filmogène lipophile comprenant du diisostearoyl polyglyceryl 3 dimer dilinoleate et
- un agent filmogène hydrophile comprenant un polyuréthane.

On entend par agent filmogène, des agents chimiques qui ne pénètrent pas dans l'épiderme et qui laissent à la surface de la peau ou du cheveu un film souple, cohésif et continu et homogène. Ils agissent en surface en renforçant ou en remplaçant le film hydrolipidique et freinent efficacement l'évaporation de l'eau. Il existe des agents filmogènes dits lipophiles, c'est-à-dire que l'ingrédient s'incorpore préférentiellement dans la phase grasse lors de la préparation de la composition. Il existe des agents filmogènes dit hydrophile, c'est-à-dire que l'ingrédient s'incorpore préférentiellement dans la phase aqueuse lors de la préparation de la composition. Les agents filmogènes les plus courants sont entre autres vinylpyrrolidone/hexadecene copolymer, triacontanyl polyvinylpyrrolidone, vinylpyrrolidone/eicosene copolymer, acrylates/octylacrylamide copolymer, acrylates copolymer, styrene/acrylates copolymer et sont majoritairement synthétiques et persistants dans l'environnement. Ces agents filmogènes courants ne présentent pas les caractéristiques requises pour obtenir le double effet technique selon l'invention.

Avantageusement, dans la composition cosmétique selon l'invention, la concentration massique en diisostearoyl polyglyceryl 3 dimer dilinoleate peut être comprise dans un intervalle allant de 0,6 à 6 %, en masse par rapport à la masse totale de la composition. De préférence la concentration massique en diisostearoyl polyglyceryl 3 dimer dilinoleate est comprise dans un intervalle allant de 1,5 % à 2,25 %, en masse par rapport à la masse totale de la composition.

On entend par diisostearoyl polyglyceryl 3 dimer dilinoleate, un composé obtenu par une réaction de condensation d'acide isostéarique et d'acide dilinoleic sur un polyglyceryl-3 obtenu par une réaction de polymérisation du glycérol. Son numéro CAS est par exemple le 187547-43-9.

Avantageusement, dans la composition cosmétique selon l'invention, la concentration massique en polyuréthane peut être comprise dans un intervalle allant de 0,5 à 5 %, en masse par rapport à la masse totale de la composition. De préférence, la concentration massique en polyuréthane est comprise dans un intervalle allant de 1 à 4,5 %, en masse par rapport à la masse totale de la composition.

On entend par polyuréthane, un composé obtenu par la polymérisation d'un isocyanante et d'un alcool. Il peut être obtenue par la réaction de polymérisation d'un copolymère d'hexanediol, de neopentyl glycol et d'acide adipique avec du diisocyanate d'hexamethylene ensuite polymérisé avec du N-(-2-aminoethyl)-2-aminoethane sulfonate de sodium et de l'éthylenediamine ou bien par la reaction de polymérisation d'un pre-polymère de polyester constitué d'acide adipique, d'hexanediol et de neopentyl glycol avec du méthylene diphényl diisocyanate saturé ou bien par la réaction de polymérisation d'un copolymère d'hexanediol, de neopentyl glycol, d'acide adipique, de diisocyanate d'isophorone et de diamine d'isophorone avec du N-(-2-aminoethyl)-2-aminoethane sulfonate de sodium, ou bien par la réaction de polymérisation d'un copolymère de 1,4-butanediol, de neopentyl glycol, d'acide succinique et de diisocyanate d'isophorone, avec du N-(-2-aminoethyl)-2-aminoethane sulfonate de sodium et du diamine d'isophorone, ou bien par la réaction de polymérisation d'un copolymère de 1 ,4-butanediol, de neopentyl glycol et d'acide succinique avec du méthylène diphényl diisocyanate saturé ensuite fonctionnalisé avec du 2-((2-aminoethyl)amino ethanesulfonate de sodium et de l'ethylenediamine. Il peut s'agir par exemple des produits commercialisés par Covestro sous le nom de Baycusan ou par Wanhua Chemical Group sous le nom de Carfil.

Avantageusement, le polyuréthane peut être choisi parmi le polyuréthane-34, le polyuréthane-35, le polyuréthane-48, le polyuréthane-93, le polyuréthane-99 et leurs mélanges. De préférence, le polyuréthane peut être choisi parmi le polyuréthane-35, le polyuréthane-99 et leurs mélanges.

Avantageusement, dans la composition cosmétique selon l'invention, la concentration massique en filtre UVA et/ou UVB peut être comprise dans un intervalle allant de 1 à 50 %, en masse par rapport à la masse totale de la composition. De préférence, la concentration massique en filtre UVA et/ou UVB est comprise dans un intervalle allant de 5 à 20 %, en masse par rapport à la masse totale de la composition. Une composition dont l'indice SPF est de 50+ aura généralement de 15 à 20 % de filtre UVA et/ou UVB. Une composition dont l'indice SPF est de 30 aura généralement de 8 à 15 % de filtre UVA et/ou UVB.

On entend par filtre UVA et/ou UVB les substances décrites à l'annexe VI du règlement (CE) no 1223/2009 du Parlement Européen et du Conseil. Il s'agit d'un composé, ou mélange de composés, organique ou inorganique qui bloque ou absorbe les radiations du spectre ultraviolet. Les filtres organiques sont des molécules organiques. Elles permettent une protection solaire en absorbant le rayonnement UV grâce à des groupements chromophores constitués de systèmes π conjugués. Lorsque ces molécules absorbent la lumière incidente elles passent dans un état excité avant d'émettre de l'énergie sous forme de chaleur ou de fluorescence pour retourner à leur état fondamental. Ces filtres se caractérisent par une longueur d'onde d'absorption maximale qui permet de les catégoriser en filtre UVA, UVB ou à champ large (UVA et UVB). C'est pourquoi pour obtenir un spectre d'action le plus large possible et une protection efficace il est nécessaire de combiner des filtres UVA et des filtres UVB. Les filtres minéraux sont généralement choisis parmi le dioxyde de titane (TiO₂) et l'oxyde de zinc (ZnO). Leur mécanisme d'action repose faiblement sur l'absorption de la lumière, mais surtout sur la diffusion de ses rayons. Ils existent sous forme pigmentaire, et laissent dans ce cas un film visible de couleur blanche sur la peau, ou sous forme nanoparticulaire (< 100 nm). Le TiO₂ est également sensible à la photodégradation, il est donc nécessaire qu'il soit enrobé afin d'éviter toute réaction photocatalytique. En plus de prévenir cette dégradation, l'enrobage du TiO₂ permet de le rendre dispersible dans le milieu d'addition. Les mêmes stratégies de formulation sont applicables au ZnO. Il est aussi possible de retrouver d'autres composés minéraux non reconnus comme des filtres UV comme le talc ou le kaolin, mais qui ont aussi un pouvoir d'absorption et/ou de dispersion de la lumière.

Le choix et les pourcentages des filtres est porté par le type de galénique désirée, la région géographique de commercialisation, le choix d'un produit conventionnel ou bio et les indices de protection visées dans l'UVA et l'UVB.

Avantageusement, le filtre UVA et/ou UVB peut être un filtre UV organique lipophile et/ou hydrophile. Le filtre UV organique lipophile et/ou hydrophile peut être un ou plusieurs filtres UV organiques lipophiles et/ou hydrophile choisis parmi les filtres UVA organiques lipophiles et/ou hydrophile, les filtres UVB organiques lipophiles et/ou hydrophile, et leurs mélanges.

Avantageusement, le filtre UVA et/ou UVB peut être choisi parmi les filtres UVA et/ou UVB organique. De préférence, le filtre UVA et/ou UVB est choisi parmi l'homosalate, l'éthylhéxyl salicylate, le bis-éthylhéxyloxyphénol méthoxyphényl triazine, l'octocrylène, le butyl méthoxydibenzoylméthane, l'éthylhéxyl triazone, le diethylamino hydroxybenzoyl hexyl benzoate, le diéthylhexyl butamido triazone, le methylene bis-benzotriazolyl tetramethylbutylphenol, le phenylbenzimidazol sulfonic acid, le disodium phenyl dibenzimidazole tetrasulfonate, ethylhexyl methoxycinnamate, isoamyl p-methoxycinnamate et leurs mélanges.
de manière encore préférée, préférence parmi le le bis-éthylhéxyloxyphénol méthoxyphényl triazine, le butyl méthoxydibenzoylméthane, l'éthylhéxyl triazone, le diethylamino hydroxybenzoyl hexyl benzoate, le diéthylhexyl butamido triazone et leurs mélanges. De manière encore préférée, le filtre UVA et/ou UVB est choisi parmi parmi le le bis-éthylhéxyloxyphénol méthoxyphényl triazine, l'éthylhéxyl triazone, le diethylamino hydroxybenzoyl hexyl benzoate, et leurs mélanges.

Avantageusement, la composition cosmétique selon l'invention peut comprendre en outre de l'eau, éventuellement un ou plusieurs éléments choisis parmi les solvants miscibles à l'eau, les corps gras, dont les huiles, les tensioactifs, les polyols, les gélifiants, les émollients, les épaississants, les ajusteurs de pH (i.e. acide ou base, organique ou inorganique), les parfums, les colorants, les pigments, les agents antimicrobiens, les conservateurs, les antioxydants, les huiles essentielles ou encore les actifs cosmétiques.

Avantageusement, la composition cosmétique selon l'invention peut comprendre en outre un ou plusieurs excipients lipophiles choisis parmi un parfum, un colorant, un pigment, une huile, de préférence d'origine végétale, un ester, un éther, un carbonate, un alcool gras, un acide gras, un antioxydant, une huile essentielle et un actif cosmétique lipophile.

On entend par excipients lipophile, lorsqu'il est liquide, un liquide non miscible à l'eau, c'est-à-dire que lorsqu'il est introduit à raison d'au moins 1% en masse dans l'eau à 25°C, n'est pas du tout soluble dans l'eau, ou soluble à hauteur de moins de 10% en masse, par rapport à la masse d'excipient introduite dans l'eau ; et lorsqu'il est solide, non soluble dans l'eau, c'est-à-dire que lorsqu'il est introduit à raison d'au moins 1% en masse dans l'eau à 25°C, n'est pas du tout soluble dans l'eau, ou soluble à hauteur de moins de 10% en masse, par rapport à la masse d'excipient introduite dans l'eau.

Avantageusement, la composition cosmétique selon l'invention peut comprendre une huile. L'huile peut être d'origine animale ou végétale, d'origine naturelle ou synthétique. L'huile est de préférence d'origine végétale et/ou de préférence d'origine naturelle.

Dans le cadre de l'invention, on entend par « huile » des composés huileux/gras dont la définition est généralement bien connue de l'homme du métier, dans le domaine technique de l'invention, qui sont liquide à température ambiante. Une huile est un liquide non miscible à l'eau, c'est-à-dire que lorsqu'il est introduit à raison d'au moins 1% en masse dans l'eau à 25°C, n'est pas du tout soluble dans l'eau, ou soluble à hauteur de moins de 10% en masse, par rapport à la masse d'huile introduite dans l'eau. Une huile peut être volatile ou non volatile. Par « non volatile », on entend une huile dont la pression de vapeur à température ambiante (25°C) et pression atmosphérique, est non nulle et inférieure à 10⁻³ mmHg (0,13 Pa). Par "volatile", on entend une huile susceptible de s'évaporer au contact de la peau en moins d'une heure, à température ambiante et pression atmosphérique. Les huiles utilisables dans les compositions cosmétiques de l'invention sont des huiles d'origine naturelle, de préférence d'origine végétale, ayant de préférence un point de fusion inférieur à 20°C.

Avantageusement, l'huile peut être choisie dans le groupe comprenant des huiles d'origine végétale, de préférence choisie parmi les huiles d'amande douce, d'avocat, de germe de blé, de noisette, de noyaux d'abricot, de ricin, de sésame, de tournesol, de riz, de coprah, de macadamia, de carthame, de jojoba, de colza, de pépins de raisin, de camélia, d'argan, de coton, de bourrache, de rosier muscat, d'onagre, d'olive, de noix, de babassu, de prune, de noix de coco, de camomille, de calendula, de menthe poivrée , de chanvre, de citron, de palme, de romarin, de sauge, de karité, de soja, de lin, du squalane (huile de sucre de canne), des alcanes en C10 à C30 et leurs mélanges.

Avantageusement, la composition cosmétique selon l'invention peut comprendre un ester (huile ester). L'ester peut être d'origine animale ou végétale, d'origine naturelle ou synthétique. L'ester est de préférence d'origine végétale et/ou de préférence d'origine naturelle.

Avantageusement, l'ester peut être choisi dans le groupe comprenant des esters, de préférence choisi parmi le phenoxytheyl caprylate, le cocoate d'éthylhexyle, le coco-caprylate/caprate, le palmitate d'isopropyle, le stéarate d'isopropyle, le stéarate d'éthylhexyle, l'isononanoate d'isononyle, le dibutyl adipate, le C12-15 alkyl benzoate, le diisopropyl sebacate, le diisobutyl adipate, isoamyl laurate, coco-caprylate, des triglycérides d'alcools en C8 à C30 (i.e. caprylic capric triglycéride) et leurs mélanges.

Avantageusement, la composition cosmétique selon l'invention peut comprendre un éther (huile éther). L'éther peut être d'origine animale ou végétale, d'origine naturelle ou synthétique. L'éther est de préférence d'origine végétale et/ou de préférence d'origine naturelle.

Avantageusement, l'éther peut être choisi dans le groupe comprenant des éthers végétaux, de préférence le dicaprylyl ether,

Avantageusement, la composition cosmétique selon l'invention peut comprendre un carbonate (huile carbonate). Le carbonate peut être d'origine animale ou végétale, d'origine naturelle ou synthétique. Le carbonate est de préférence d'origine végétale et/ou de préférence d'origine naturelle.

Avantageusement, le carbonate peut être choisi dans le groupe comprenant le dicaprylyl carbonate, le butylène carbonate, le 14-15 dialkyl carbonate, diethyl carbonate, dipropyl carbonate, dipropylheptyl carbonate, propylene carbonate et leurs mélanges.

Avantageusement, l'acide gras peut être choisi dans le groupe comprenant des acides gras végétaux, de préférence choisi parmi les acides gras en en C8 à C26. De préférence, l'acide gras peut être choisi parmi l'acide laurique, l'acide cétylique, l'acide cétéarylique, l'acide stéarique, l'acide palmitique, l'acide myristique, l'acide béhénique, l'acide arachidique et leurs mélanges.

Avantageusement, l'alcool gras peut être choisi dans le groupe comprenant des alcool gras végétaux, de préférence choisi parmi les alcools gras en C10 à C22. De préférence, l'alcool gras peut être choisi parmi l'alcool cétylique, l'alcool cétéarylique, l'alcool palmitique, l'alcool stéarique, l'alcool béhénique, l'alcool arachidique et leurs mélanges.

Avantageusement, la composition cosmétique selon l'invention peut comprendre en outre au moins un pigment et/ou colorant. Le pigment peut être choisi dans le groupe comprenant les pigments organiques, inorganiques et/ou composites tels que par exemples les pigments monochromes (blanc, noir ou colorés), les laques, les nacres et/ou les pigments à effet d'optique. Ils peuvent être synthétiques ou naturels.

Dans le cadre de l'invention, on entend par « pigment », des particules organiques, inorganiques ou composites, qui peuvent être monochromes, des laques, des nacres, ou des pigments à effet optiques, comme les pigments réfléchissants et les pigments goniochromatiques. Il peut s'agir par exemple d'un pigment tels que ceux décrits dans le règlement (CE) no 1223/2009 du Parlement Européen et du Conseil. Les pigments minéraux (inorganiques) peuvent être choisis parmi les pigments d'oxydes métalliques (tels que les oxydes de cérium, chrome, fer, titane, zinc ou zirconium, le violet de manganèse, le bleu de prusse, le bleu outremer, le bleu ferrique, et leurs mélanges). Les pigments organiques peuvent être D&C red 6, D&C red 27 aluminium lake, FD&C red 40 aluminium lake, D&C red 21 aluminium lake, D&C red 36, FD&C yellow 5 aluminium lake, FDC Blue 1 aluminium lake, D&C red 7 aluminium lake, D&C red 30.

Dans le cadre de l'invention, on entend par « nacre », des particules ou éléments colorées de toutes formes, irisées ou non, naturels ou synthétiques, qui peuvent être produites dans leur coquille ou bien synthétisées. Les nacres présentent généralement des effets de couleurs. Les nacres peuvent être créées sur différents supports dont le mica, le fluorflogopithe synthétique, l'oxychlorure de bismuth et le borosilicate et associées aux pigments listés au paragraphe précédent.

Dans le cadre de l'invention, on entend par « colorant », des substances colorantes qui ne sont pas des pigments ou des nacres. Il peut s'agir par exemple d'un colorant tels que ceux décrits dans le règlement (CE) no 1223/2009 du Parlement Européen et du Conseil.

Avantageusement, la composition cosmétique selon l'invention peut comprendre en outre au moins un parfum. On entend par « parfum » une composition parfumante dont la fonction première consiste à modifier l'odeur d'un produit ou impartir une odeur à un produit.

Avantageusement, la composition cosmétique selon l'invention peut comprendre en outre au moins un actif cosmétique lipophile.

Dans le cadre de l'invention, on entend par « actif cosmétique », une substance ou un mélange de substances destinée(s) à être mis en contact avec les parties superficielles du corps humain (plus particulièrement l'épiderme, les systèmes pileux et capillaire, les lèvres, etc.) ou avec les dents et les muqueuses buccales, en vue, exclusivement ou principalement, de les nettoyer, de les parfumer, d'en modifier l'aspect, de les protéger, de les maintenir en bon état ou de corriger les odeurs corporelles. Un actif cosmétique est ainsi généralement un ingrédient qui agit sur la peau pour lui procurer un bienfait réel. Par exemple, un actif peut être nourrissant, apaisant, hydratant, matifiant, redensifiant.

Avantageusement, l'actif cosmétique lipophile peut être choisi dans le groupe comprenant un extrait de bleuet, de matricaire, de camomille, de romarin, de calendula. L'actif cosmétique peut être facilement sélectionné par l'homme du métier en fonction de l'effet cosmétique recherché (anti-âge, nourrissant, apaisant, hydratant, matifiant, redensifiant, déodorant).

Avantageusement, la composition cosmétique selon l'invention peut comprendre en outre au moins une huile essentielle. On entend par « huile essentielle », un liquide huileux concentré en substances végétales, obtenu par extraction ou distillation de molécules volatiles de la plante d'origine. On retrouve majoritairement des terpénoïdes et des molécules aromatiques. Les huiles essentielles issues de différentes plantes possèdent donc des propriétés différentes, dépendantes de la composition d'origine.

Avantageusement, la composition cosmétique selon l'invention peut comprendre en outre un ou plusieurs excipients hydrophiles choisis parmi un tensioactif, un colorant, un polyol, un gélifiant (i.e. un polysaccharide, dont notamment les gommes, et/ou un carbomer), un agent régulateur de pH (i.e. acide ou base, organique ou inorganique), un conservateur, un agent antimicrobien, un agent chélatant et un actif cosmétique hydrophile.

Avantageusement, le pH de la composition peut être compris dans un intervalle allant de 5 à 8. La nature et la quantité de l'agent régulateur de pH peut être choisi en fonction de ce paramètre. L'agent régulateur de pH peut par exemple être de la soude (NaOH), un sel d'arginine, de l'acide citrique ou de l'acide lactique.

On entend par excipient hydrophile, un liquide miscible à l'eau, c'est-à-dire que lorsqu'il est introduit à raison d'au moins 0,1 % en masse dans l'eau à 25°C, est complètement soluble dans l'eau, ou soluble à hauteur d'au moins 90% en masse, par rapport à la masse d'excipient introduite dans l'eau ; et lorsqu'il est solide, soluble dans l'eau, c'est-à-dire soluble au moins de 0,1 g/L.

Avantageusement, la composition selon l'invention peut comprendre en outre au moins un tensioactif. Le tensioactif peut être un tensioactif anionique, cationique, non-ionique, amphotère ou une association de leurs mélanges.

Avantageusement, le tensioactif anionique peut être choisi dans le groupe comprenant un alkyl sulfate, un alkyl sulfoacétate, un iséthionate, un taurate, un acylglutamate, et un sarcosinate.

Avantageusement, la composition selon l'invention peut comprendre un, ou deux, ou trois des tensio-actifs anioniques cités. Par exemple, la composition de l'invention peut contenir un ammonium lauryl sulfate, un lauryl sulfoacétate, un sodium cocoyl isethionate, un sodium lauroyl methyl isethionate, un sodium methyl cocoyl taurate, un sodium lauroyl sarcosinate, un sodium stearoyl glutamate, un sodium lauroyl glutamate, disodium cetearyl sulfosuccinate, un sodium lauryl glucose carboxylate ou un mélange.

Selon l'invention, le au moins un tensio-actif amphotère peut être choisi dans le groupe comprenant les N-alkylamidobétaïnes, les bétaïnes, les sultaïnes, les alkylpolyaminocarboxylates, les alkylamphoacétates, leurs dérivés, et les dérivés de la glycine. Par exemple la composition selon l'invention peut contenir la bétaïne, la cocamidopropyl betaïne, la cocobetaïne, la cocamidopropyl hydroxysultaine, le sodium cocoamphoacetate.

Selon l'invention, le au moins un tensio-actif non-ionique peut être choisi dans le groupe comprenant les glycolipides, les alkypolyglucosides, les esters de glycéryle et d'acide gras, les esters de sucrose et d'acides gras, les esters de sucrose oxyalkylénés, les esters de glycérol oxyalkylénés, les esters d'acides gras et de polyéthylène glycol, les esters d'acide gras et de sorbitan, les alcools gras polyglycérolés et les dérivés de glucamine. Par exemple : la composition de l'invention peut contenir le décylglucoside, le laurylglucoside, le glyceryl oleate, le pEG-7 glyceryl cocoate, le glyceryl stéarate citrate, le sorbitan palmitate, le cocoyl methyl glucamide, polyglyceryl-3 dicitrate/stearate le polyglyceryl-6 caprylate, le polyglyceryl-4 caprate, le polyglyceryl-6 polyricinoleate, le polyglyceryl-3 ricinoleate, le polyglyceryl-2 dipolyhydroxysteate.

Avantageusement, la composition cosmétique selon l'invention peut comprendre en outre au moins un gélifiant. Le gélifiant peut être un polysaccharide, un carbomer (par exemple de CAS = 9007-20-9 / 9003-01-4 / 76050-42-5 / 9062-04-8) ou un polyacrylate, de préférence un polysaccharide. Le polysaccharide peut être choisi parmi les gommes telles que la gomme xanthane, la gomme diutane, la gomme de konjac ou la gomme gellane, l'alginate, le glucomannane, le chondrus crispus powder, le carraghénane, l'acide hyaluronique (ex : sodium hyaluronate), l'amidon, le sodium carboxymethyl starch, la cellulose, la cellulose microcristalline, et leurs mélanges. De préférence, le gélifiant est choisi parmi la gomme xanthane (par exemple de CAS 11138-66-2), la gomme diutane, le glucomannane, le sodium carboxymethyl starch, l'alginate, le chondrus crispus powder, la cellulose, la cellulose microcristalline et leurs mélanges.

Avantageusement, la composition cosmétique selon l'invention peut comprendre en outre au moins un polyol, de préférence un polyol en C3 à C10. On entend par polyol, un composé hydrocarboné comprenant au moins 2 fonctions hydroxyle. Le polyol en C3 à C10 peut être linéaire ou ramifié. Le polyol peut être choisi parmi le glycerol, le diglycerol, le propylène glycol (propan-1,2-diol), le butylène glycol (butan-1,2-diol), le 1,4-butanediol, le pentylène glycol (pentan-1,2-diol), le 1,5-pentanediol, le propane-1,3-diol, le méthypropanediol, le 1,2-hexanediol, le 1,2-heptanediol, le butyl ethyl propanediol, le 1,2-octanediol, le 1,2-decanediol le caprylyl glycol et leurs mélanges. De préférence, le polyol est choisi parmi le glycérol, le propylène glycol, le pentylène glycol, le caprylyl glycol, le 1,2-hexanediol et leurs mélanges.

Avantageusement, la composition cosmétique selon l'invention peut comprendre en outre au moins un conservateur et/ou un agent antimicrobien. Le au moins un conservateur et/ou un agent antimicrobien peut être choisi parmi les substances listées dans l'Annexe V « Conservateurs » du règlement cosmétiques (CE) n° 1223/2009 du 30 novembre 2009 peuvent être ajoutées, et plus particulièrement parmi l'acide sorbique et ses sels, l'acide salicylique et ses sels, l'acide benzoïque, ses sels et ses esters, l'acide déhydroacétique et ses sels, le phénylpropanol, l'alcool benzylique et leurs mélanges.

Avantageusement, la composition cosmétique selon l'invention peut comprendre en outre au moins un antioxydant. L'antioxydant peut être choisi parmi l'hydroxyacetophenone, le tocopheryl acetate, le hydroxymethoxyphenyl decanone, le tocophérol et leurs mélanges. Il peut s'agir de toute autre substance capable de ralentir ou empêcher l'oxydation de la composition cosmétique.

Avantageusement, la composition cosmétique selon l'invention peut comprendre en outre au moins un actif cosmétique hydrophile.

Avantageusement, l'actif cosmétique hydrophile peut être choisi dans le groupe comprenant une eau florale, un extrait de plante ou un extrait d'algue telle que centaurea cyanus flower water (Eau florale de bleuet) ou carpobrotus edulis extract ou porphyridium extract.

Avantageusement, la composition cosmétique selon l'invention peut comprendre en outre au moins un agent chélatant. L'agent chélatant peut être choisi parmi le trisodium ethylenediamine disuccinate, le sodium phytate, le sodium gluconate, le tetrasodium glutamate diacetate, le gluconate de sodium, le gluconate de potassium, l'acide caprylhydroxamique, l'acide phytique et leurs mélanges. Il peut s'agir de toute autre substance cosmétique ayant la capacité de fixer les ions positifs pour former un complexe stable.

Avantageusement, la composition cosmétique selon l'invention peut comprendre en outre un ou plusieurs sels organiques ou inorganiques, un ou plusieurs acide organiques, tels que le chlorure de sodium, le chlorure de calcium, le chlorure de potassium, l'hydroxyde de sodium (ajusteur de pH), l'acide citrique, le sodium bicarbonate, l'acide lactique, le sodium citrate et leurs mélanges.

Avantageusement, la composition cosmétique selon l'invention peut comprendre en outre des vitamines, telles que la vitamine C, l'inositol (B7), le panthenol (B5), Vitamine F (ethyl linoleate), vitamine E (tocopherol), vitamine A (retinol).

Avantageusement, la composition cosmétique selon l'invention peut être sous forme d'une émulsion huile-dans-eau. Il peut s'agir d'une crème, d'un lait, d'un spray, d'une émulsion coulée et/ou d'une émulsion colorée telle qu'une BB crème ou un fond de teint. La composition cosmétique selon l'invention peut être sous forme d'une émulsion eau-dans-huile. Il peut s'agir d'une crème, d'un lait, d'un spray, d'une émulsion coulée et/ou d'une émulsion colorée telle qu'une BB crème ou un fond de teint.

Avantageusement, la composition cosmétique selon l'invention peut être une émulsion huile-dans-eau, sous la forme d'un lait SPF50+, et comprendre :
- de 0,6 à 6 % de diisostearoyl polyglyceryl 3 dimer dilinoleate,
- de 0,5 à 5 % de polyuréthane,
- de 6 à 10 % d'un filtre UVA choisi parmi le diethylamino hydroxybenzoyl hexyl benzoate, le butyl méthoxydibenzoylméthane, le disodium phenyl dibenzimidazole tetrasulfonate et leurs mélanges,
- de 6 à 20 % d'un filtre UVB choisi parmi l'homosalate, l'éthylhéxyl salicylate, le bis-éthylhéxyloxyphénol méthoxyphényl triazine, l'octocrylène, l'éthylhexyl triazone, le diéthylhexyl butamido triazone, le methylene bis-benzotriazolyl tetramethylbutylphenol, le phenylbenzimidazol sulfonic acid, ethylhexyl methoxycinnamate, isoamyl p-methoxycinnamate et leurs mélanges,
- de 16 à 30 % d'une huile parmi dicaprylyl carbonate, le dibutyl adipate, le caprylic / capric triglycéride, le phenoxytheyl caprylate, le c12-15 alkyl benzoate et leurs mélanges,
- de 0,01 à 5 % gélifiant choisi parmi la gomme xanthane, le glucomannane, la gomme gellane, le carraghénane, la gomme cellulose, la cellulose microcristalline, la gomme arabique, la gomme diutane et leurs mélanges,
- de 0,5 à 4 % d'un tensioactif choisi parmi le lauryl glucoside, le polyglyceryl-2 dipolyhydroxysteate, le glyceryl stéarate citrate, le polyglyceryl-3 dicitrate/stearate, le sodium lauryl glucose carboxylate, le disodium cetearyl sulfosuccinate, le sodium stearoyl glutamate, le sodium lauroyl glutamate, le polyglyceryl-6-polyricinoleate et leurs mélanges,
- de 0,5 à 4 % d'acide gras et/ou d'alcool gras choisi parmi l'alcool stéarique , l'alcool cétéarylique, l'alcool cétylique, l'alcool béhénique, l'alcool arachidique, l'acide stéarique, l'acide cétéarique, l'acide cétylique, l'acide béhénique, l'acide arachidique et leurs mélanges,
- de 0,01 à 4 % de polyol parmi le glycérol, le propylène glycol, le pentylène glycol, l'hexylène glycol, le caprylyl glycol, le 1,2-hexanediol, le 1,2-heptanediol, et leurs mélanges,
- optionnellement de 0,01 à 4 % d'antioxydant parmi l'hydroxyacetophenone, le tocopheryl acetate, le tocopherol et leurs mélanges,
- optionnellement de 0,01 à 2 % d'agent chélatant parmi le trisodium ethylenediamine disuccinate, le sodium phytate, le sodium gluconate, le gluconate de sodium, de gluconate de potassium, le tetrasodium glutamate diacetate, le caprylhydroxamic acid, le phytic acid et leurs mélanges,
- de 15 à 70 % d'eau,
les pourcentages étant donnés par rapport à la masse totale (100%) de la composition.

Avantageusement, la composition cosmétique selon l'invention peut être une émulsion huile-dans-eau, sous la forme d'un lait SPF50+, et comprendre :
- de 1,5 à 2,25 % de diisostearoyl polyglyceryl 3 dimer dilinoleate,
- de 1 à 4,5 % de polyuréthane,
- de 6 à 8 % d'un filtre UVA choisi parmi le diethylamino hydroxybenzoyl hexyl benzoate, le butyl méthoxydibenzoylméthane, le disodium phenyl dibenzimidazole tetrasulfonate et leurs mélanges,
- de 6 à 15 % d'un filtre UVB choisi parmi l'homosalate, l'éthylhéxyl salicylate, le bis-éthylhéxyloxyphénol méthoxyphényl triazine, l'octocrylène, l'éthylhexyl triazone, le diéthylhexyl butamido triazone, le methylene bis-benzotriazolyl tetramethylbutylphenol, le phenylbenzimidazol sulfonic acid, ethylhexyl methoxycinnamate, isoamyl p-methoxycinnamate et leurs mélanges,
- de 16 à 25 % d'une huile parmi dicaprylyl carbonate, le dibutyl adipate, le caprylic / capric triglycéride, le phenoxytheyl caprylate, le c12-15 alkyl benzoate et leurs mélanges,
- de 0,01 à 2 % gélifiant choisi parmi la gomme xanthane, le glucomannane, la gomme gellane, le carraghénane, la gomme cellulose, la cellulose microcristalline, la gomme arabique, la gomme diutane et leurs mélanges,
- de 1 à 4 % d'un tensioactif choisi parmi le lauryl glucoside, le polyglyceryl-2 dipolyhydroxysteate, le glyceryl stéarate citrate, le polyglyceryl-3 dicitrate/stearate, le sodium lauryl glucose carboxylate, le disodium cetearyl sulfosuccinate, le sodium stearoyl glutamate, le sodium lauroyl glutamate, le polyglyceryl-6-polyricinoleate et leurs mélanges,
- de 0,5 à 3 % d'acide gras et/ou d'alcool gras choisi parmi l'alcool stéarique , l'alcool cétéarylique, l'alcool cétylique, l'alcool béhénique, l'alcool arachidique, l'acide stéarique, l'acide cétéarique, l'acide cétylique, l'acide béhénique, l'acide arachidique et leurs mélanges,
- de 0,5 à 4 % de polyol parmi le glycérol, le propylène glycol, le pentylène glycol, l'hexylène glycol, le caprylyl glycol, le 1,2-hexanediol, le 1,2-heptanediol, et leurs mélanges,
- optionnellement de 0,1 à 2 % d'antioxydant parmi l'hydroxyacetophenone, le tocopheryl acetate, le tocopherol et leurs mélanges,
- optionnellement de 0,01 à 1 % d'agent chélatant parmi le trisodium ethylenediamine disuccinate, le sodium phytate, le sodium gluconate, le gluconate de sodium, de gluconate de potassium, le tetrasodium glutamate diacetate, le caprylhydroxamic acid, le phytic acid et leurs mélanges,
- de 40 à 70 % d'eau,
les pourcentages étant donnés par rapport à la masse totale (100%) de la composition.

Avantageusement, la composition cosmétique selon l'invention peut être une émulsion huile-dans-eau, sous la forme d'un lait SPF30, et comprendre :
- de 0,5 à 6 % de diisostearoyl polyglyceryl 3 dimer dilinoleate,
- de 0,5 à 5 % polyuréthane,
- de 1 à 6 % d'un filtre UVA choisi parmi le diethylamino hydroxybenzoyl hexyl benzoate, le butyl méthoxydibenzoylméthane et leurs mélanges,

- de 2 à 6 % d'un filtre UVB choisi parmi le bis-éthylhéxyloxyphénol méthoxyphényl triazine, l'éthylhexyl triazone, le diéthylhexyl butamido triazone et leurs mélanges,
- de 5 à 16 % d'une huile parmi dicaprylyl carbonate, le dibutyl adipate, le caprylic / capric triglycéride, le phenoxytheyl caprylate, le C12-15 alkyl benzoate et leurs mélanges,
- de 0,01 à 5 % d'un agent gélifiant choisi parmi la gomme xanthane, la gomme cellulose, la cellulose microcristalline, la gomme diutane et leurs mélanges,
- de 0,5 à 4 % d'un tensioactif choisi parmi le lauryl glucoside, le polyglyceryl-2 dipolyhydroxysteate, le glyceryl stéarate citrate, le polyglyceryl-3 dicitrate/stearate, le sodium lauryl glucose carboxylate, le disodium cetearyl sulfosuccinate, le sodium stearoyl glutamate, le sodium lauroyl glutamate, le polyglyceryl-6-polyricinoleate et leurs mélanges,
- de 0,5 à 4 % d'acide gras et/ou d'alcool gras choisi parmi l'alcool stéarique , l'alcool cétéarylique, l'alcool cétylique, l'alcool béhénique, l'alcool arachidique, l'acide stéarique, l'acide cétéarique, l'acide cétylique, l'acide béhénique, l'acide arachidique et leurs mélanges.,
- de 0,01 à 4 % de polyol parmi, le pentylène glycol, le caprylyl glycol, le 1,2-hexanediol et leurs mélanges,
- optionnellement de 0,01 à 4 % d'antioxydant parmi l'hydroxyacetophenone, le tocopheryl acetate et leurs mélanges,
- optionnellement de 0,01 à 2 % d'agent chélatant parmi le trisodium ethylenediamine disuccinate, le sodium phytate, le sodium gluconate, le gluconate de sodium, de gluconate de potassium, le tetrasodium glutamate diacetate, le caprylhydroxamic acid, le phytic acid et leurs mélanges,
- de 34 à 86 % d'eau,
les pourcentages étant donnés par rapport à la masse totale (100%) de la composition.

Avantageusement, la composition cosmétique selon l'invention peut être une émulsion huile-dans-eau, sous la forme d'un lait SPF30, et comprendre :
- de 1,5 à 2,5 % de diisostearoyl polyglyceryl 3 dimer dilinoleate,
- de 1 à 4,5 % polyuréthane,
- de 2 à 6 % d'un filtre UVA choisi parmi le diethylamino hydroxybenzoyl hexyl benzoate, le butyl méthoxydibenzoylméthane et leurs mélanges,
- de 3 à 6 % d'un filtre UVB choisi parmi le bis-éthylhéxyloxyphénol méthoxyphényl triazine, l'éthylhexyl triazone, le diéthylhexyl butamido triazone et leurs mélanges,
- de 8 à 12 % d'un d'une huile parmi dicaprylyl carbonate, le dibutyl adipate, le caprylic / capric triglycéride, le phenoxytheyl caprylate, le C12-15 alkyl benzoate et leurs mélanges,
- de 0,01 à 2 % d'un agent gélifiant choisi parmi la gomme xanthane, la gomme cellulose, la cellulose microcristalline, la gomme diutane et leurs mélanges
- de 1 à 4 % d'un tensioactif choisi parmi le lauryl glucoside, le polyglyceryl-2 dipolyhydroxysteate, le glyceryl stéarate citrate, le polyglyceryl-3 dicitrate/stearate, le sodium lauryl glucose carboxylate, le disodium cetearyl sulfosuccinate, le sodium stearoyl glutamate, le sodium lauroyl glutamate, le polyglyceryl-6-polyricinoleate et leurs mélanges,
- de 0,5 à 3% d'acide gras et/ou d'alcool gras choisi parmi l'alcool stéarique , l'alcool cétéarylique, l'alcool cétylique, l'alcool béhénique, l'alcool arachidique, l'acide stéarique, l'acide cétéarique, l'acide cétylique, l'acide béhénique, l'acide arachidique et leurs mélanges.,
- de 0,5 à 4% de polyol parmi, le pentylène glycol, le caprylyl glycol, le 1,2-hexanediol et leurs mélanges,
- optionnellement de 0,1 à 2% d'antioxydant parmi l'hydroxyacetophenone, le tocopheryl acetate et leurs mélanges,
- optionnellement de 0,01 à 1% d'agent chélatant parmi le trisodium ethylenediamine disuccinate, le sodium phytate, le sodium gluconate, le gluconate de sodium, de gluconate de potassium, le tetrasodium glutamate diacetate, le caprylhydroxamic acid, le phytic acid et leurs mélanges,
- de 50 à 84 % d'eau,
les pourcentages étant donnés par rapport à la masse totale (100%) de la composition.

Avantageusement, la composition cosmétique selon l'invention peut être une émulsion eau-dans-huile, sous la forme d'un lait SPF50+, et comprendre :
- de 0,6 à 6 % de diisostearoyl polyglyceryl 3 dimer dilinoleate,
- de 0,5 à 5 % de polyuréthane,
- de 6 à 10 % d'un filtre UVA choisi parmi le diethylamino hydroxybenzoyl hexyl benzoate, le butyl méthoxydibenzoylméthane, le disodium phenyl dibenzimidazole tetrasulfonate et leurs mélanges,
- de 6 à 20 % d'un filtre UVB choisi parmi l'homosalate, l'éthylhéxyl salicylate, le bis-éthylhéxyloxyphénol méthoxyphényl triazine, l'octocrylène, l'éthylhexyl triazone, le diéthylhexyl butamido triazone, le methylene bis-benzotriazolyl tetramethylbutylphenol, le phenylbenzimidazol sulfonic acid, ethylhexyl methoxycinnamate, isoamyl p-methoxycinnamate et leurs mélanges,
- de 16 à 30 % d'une huile parmi dicaprylyl carbonate, le dibutyl adipate, le caprylic / capric triglycéride, le phenoxytheyl caprylate, le c12-15 alkyl benzoate et leurs mélanges,
- de 0,01 à 5 % gélifiant choisi parmi la gomme xanthane, le glucomannane, la gomme gellane, le carraghénane, la gomme cellulose, la cellulose microcristalline, la gomme arabique, la gomme diutane et leurs mélanges,
- de 0,5 à 4 % d'un tensioactif choisi parmi le lauryl glucoside, le polyglyceryl-2 dipolyhydroxysteate, le glyceryl stéarate citrate, le polyglyceryl-3 dicitrate/stearate, le sodium lauryl glucose carboxylate, le disodium cetearyl sulfosuccinate, le sodium stearoyl glutamate, le sodium lauroyl glutamate, le polyglyceryl-6-polyricinoleate et leurs mélanges,
- de 0,5 à 15 % d'acide gras et/ou d'alcool gras choisi parmi l'alcool stéarique , l'alcool cétéarylique, l'alcool cétylique, l'alcool béhénique, l'alcool arachidique, l'acide stéarique, l'acide cétéarique, l'acide cétylique, l'acide béhénique, l'acide arachidique et leurs mélanges,
- de 0,01 à 4 % de polyol parmi le glycérol, le propylène glycol, le pentylène glycol, l'hexylène glycol, le caprylyl glycol, le 1,2-hexanediol, le 1,2-heptanediol, et leurs mélanges,
- optionnellement de 0,01 à 4 % d'antioxydant parmi l'hydroxyacetophenone, le tocopheryl acetate, le tocopherol et leurs mélanges,
- optionnellement de 0,01 à 2 % d'agent chélatant parmi le trisodium ethylenediamine disuccinate, le sodium phytate, le sodium gluconate, le gluconate de sodium, de gluconate de potassium, le tetrasodium glutamate diacetate, le caprylhydroxamic acid, le phytic acid et leurs mélanges,
- de 15 à 70 % d'eau,
les pourcentages étant donnés par rapport à la masse totale (100%) de la composition.

Avantageusement, la composition cosmétique selon l'invention peut être une émulsion eau-dans-huile, sous la forme d'un lait SPF50+, et comprendre :
- de 1,5 à 2,25 % de diisostearoyl polyglyceryl 3 dimer dilinoleate,
- de 1 à 4,5 % de polyuréthane,
- de 6 à 8 % d'un filtre UVA choisi parmi le diethylamino hydroxybenzoyl hexyl benzoate, le butyl méthoxydibenzoylméthane, le disodium phenyl dibenzimidazole tetrasulfonate et leurs mélanges,
- de 6 à 15 % d'un filtre UVB choisi parmi l'homosalate, l'éthylhéxyl salicylate, le bis-éthylhéxyloxyphénol méthoxyphényl triazine, l'octocrylène, l'éthylhexyl triazone, le diéthylhexyl butamido triazone, le methylene bis-benzotriazolyl tetramethylbutylphenol, le phenylbenzimidazol sulfonic acid, ethylhexyl methoxycinnamate, isoamyl p-methoxycinnamate et leurs mélanges,
- de 16 à 25 % d'une huile parmi dicaprylyl carbonate, le dibutyl adipate, le caprylic / capric triglycéride, le phenoxytheyl caprylate, le c12-15 alkyl benzoate et leurs mélanges,
- de 0,01 à 2 % gélifiant choisi parmi la gomme xanthane, le glucomannane, la gomme gellane, le carraghénane, la gomme cellulose, la cellulose microcristalline, la gomme arabique, la gomme diutane et leurs mélanges,
- de 3 à 10 % d'un tensioactif choisi parmi le lauryl glucoside, le polyglyceryl-2 dipolyhydroxysteate, le glyceryl stéarate citrate, le polyglyceryl-3 dicitrate/stearate, le sodium lauryl glucose carboxylate, le disodium cetearyl sulfosuccinate, le sodium stearoyl glutamate, le sodium lauroyl glutamate, le polyglyceryl-6-polyricinoleate et leurs mélanges
- de 0,5 à 3 % d'acide gras et/ou d'alcool gras choisi parmi l'alcool stéarique , l'alcool cétéarylique, l'alcool cétylique, l'alcool béhénique, l'alcool arachidique, l'acide stéarique, l'acide cétéarique, l'acide cétylique, l'acide béhénique, l'acide arachidique et leurs mélanges,
- de 0,5 à 4 % de polyol parmi le glycérol, le propylène glycol, le pentylène glycol, l'hexylène glycol, le caprylyl glycol, le 1,2-hexanediol, le 1,2-heptanediol, et leurs mélanges,
- optionnellement de 0,1 à 2 % d'antioxydant parmi l'hydroxyacetophenone, le tocopheryl acetate, le tocopherol et leurs mélanges,
- optionnellement de 0,01 à 1 % d'agent chélatant parmi le trisodium ethylenediamine disuccinate, le sodium phytate, le sodium gluconate, le gluconate de sodium, de gluconate de potassium, le tetrasodium glutamate diacetate, le caprylhydroxamic acid, le phytic acid et leurs mélanges,
- de 40 à 70 % d'eau,
les pourcentages étant donnés par rapport à la masse totale (100%) de la composition.

De manière surprenante, les compositions cosmétiques selon l'invention permettent d'obtenir, notamment grâce à l'association d'agent filmogène lipophile comprenant du diisostearoyl polyglyceryl 3 dimer dilinoleate et d'agent filmogène hydrophile comprenant un polyuréthane selon l'invention, les avantages suivants :
- améliorer la protection solaire d'une composition de protection solaire in vivo par rapport à une composition identique excepté qu'elle ne comporte pas d'agent filmogène,
- améliorer la résistance à l'eau jusqu'à obtenir un facteur de protection solaire résiduel supérieur à 50 % après immersion de deux bains de 20 minutes en conditions normées et un facteur de protection solaire résiduel supérieur à 50 % après immersion de quatre bains de 20 minutes en conditions normées.

Dans la présente invention, lorsqu'un intervalle est donné, les bornes sont comprises. Par exemple, pour un intervalle tel que « allant de 1 à 5 % », les valeurs 1 % et 5 % sont comprises dans l'intervalle.

Dans la présente invention, sauf mention contraire, tous les pourcentages (%) sont donnés en pourcentage massique, par rapport à la masse totale de la composition cosmétique. Dans le cas de compositions cosmétiques telles que des compositions dites biphasiques ou d'émulsions, les pourcentages peuvent aussi être donnés, lorsque cela est indiqué, par rapport à la masse totale de la phase huileuse ou de la phase aqueuse.

En dehors des filtres UVA et/ou UVB, les compositions cosmétiques selon l'invention sont exclusivement ou quasiment exclusivement composées de produits d'origine naturelle. Ainsi, une composition selon l'invention comprend généralement moins de 50 % de produits d'origine synthétique par rapport à la masse totale de la composition, de préférence moins de 40 %, voire moins de 35 %. Sont désignées comme matière d'origine synthétique les composés inévitables tels que les filtres UV, certaines huiles, corps gras ou solvants desdits filtres UV, les parfums, les conservateurs, certaines charges et les pigments indispensables dans les produits de maquillage (i.e fond de teint). Une composition de type protection solaire selon l'invention peut comprendre généralement moins de 50 % (≤ 50 %) de produits d'origine synthétique (i.e. les filtres UVA et/ou UVB, les huiles, les corps gras, les conservateurs, ou les parfums). Par exemple, une composition de protection solaire de type émulsion huile-dans-eau SPF 50+, peut comprendre généralement moins de 35 % de produits d'origine synthétique. Par exemple, une composition de protection solaire de type huile dans eau SPF30 peut comprendre généralement moins de 30 % de produits d'origine synthétique.

Dans le cadre de l'invention, on entend par « produit d'origine naturelle », un produit entrant dans la définition donnée par la norme ISO 16128 : les ingrédients dérivés de matériaux naturels sont des ingrédients cosmétiques, pour une proportion de plus de 50 % d'origine naturelle, par leur masse moléculaire, selon leur teneur en carbone renouvelable, ou selon toute autre méthode appropriée, qui sont obtenus par des procédés chimiques et/ou biologiques définis visant à les modifier chimiquement. Il peut s'agir d'un composé chimique ou d'une substance chimique produite par un organisme vivant, c'est-à-dire qui se trouve dans la nature. Dans le cadre de l'invention, on considère comme produit d'origine naturelle des produits tels que les beurres ou huiles issus des végétaux ou d'hydrogénation d'huiles d'origine naturelle, de préférence végétales.

Avantageusement, la composition cosmétique selon l'invention peut comprendre en outre un ou plusieurs excipients cosmétiques supplémentaires utilisés classiquement en cosmétique, tel qu'une cire, un beurre ou tout autre composé classiquement utilisé en cosmétique, de préférence d'origine naturelle.

Avantageusement, la composition cosmétique selon l'invention comprend plusieurs phases (dont au moins une phase aqueuse). La composition selon l'invention peut être sous forme sous forme d'une émulsion huile dans eau (telle qu'une crème, un lait, un spray, une émulsion coulée et/ou d'une émulsion colorée telle qu'une BB crème, un fond de teint), d'une émulsion eau dans huile (telle qu'une crème, un lait, un spray, une émulsion coulée et/ou d'une émulsion colorée telle qu'une BB crème, fond de teint), d'une composition bi-phasique (i.e. une composition comprenant une phase grasse ou huileuse et une phase aqueuse où les deux phases sont optiquement séparées à une vitesse de cisaillement nulle et deviennent homogènes par agitation et reviennent à nouveau à deux phases séparées après l'arrêt de l'agitation).

L'invention comprend aussi une utilisation d'une combinaison d'un agent filmogène lipophile comprenant du diisostearoyl polyglyceryl 3 dimer dilinoleate et d'un agent filmogène hydrophile comprenant un polyuréthane dans une composition cosmétique de protection solaire. La composition cosmétique de protection solaire peut se présenter sous la forme d'une émulsion huile dans eau (telle qu'une crème, un lait, un spray, et/ou d'une émulsion colorée telle qu'une BB crème, un fond de teint) et/ou d'une émulsion eau dans huile (telle qu'une crème, un lait, un spray, et/ou d'une émulsion colorée telle qu'une BB crème, fond de teint), d'une composition bi-phasique (i.e. une composition comprenant une phase grasse ou huileuse et une phase aqueuse où les deux phases sont optiquement séparées à une vitesse de cisaillement nulle et deviennent homogènes par agitation et reviennent à nouveau à deux phases séparées après l'arrêt de l'agitation).

L'invention se rapporte également à un procédé cosmétique de protection solaire des matières kératiniques (peau et/ou cheveux), de préférence les lèvres et/ou de la peau (visage et/ou corps), comprenant au moins une étape consistant à appliquer sur les lèvres, la peau (visage et/ou corps) et/ou les cheveux, au moins une composition cosmétique selon l'invention.

Avantageusement, l'utilisation selon l'invention peut être une utilisation de la composition cosmétique selon l'invention pour protéger les matières kératiniques (peau et/ou cheveux), de préférence les lèvres et/ou la peau (visage et/ou corps) du soleil, en particulier des UVA et/ou UVB.

### Exemples

L'invention est davantage illustrée par les exemples suivants, de manière non limitative.

### Exemple 1 : compositions de protection solaire selon l'invention

Les compositions de référence (hors invention), L1 (hors invention), LH1, LH2, LH3, LH4 et H4 (hors invention).

**[Table 1]**

| INCI | PHASE | Référence (hors invention) | L1 (hors invention) | LH1 | LH2 |
|---|---|---|---|---|---|
| AQUA | A | 45-65 | 45-65 | 45-65 | 38,78 |
| POTASSIUM GLUCONATE | A | 0,01-0,5 | 0,01-0,5 | 0,01-0,5 | - |
| SODIUM HYDROXYDE | A | - | - | - | 0,22 |
| PHENYLBENZIMIDAZOLE SULFONIC ACIDE | A | - | - | - | 1 |
| PENTYLENE GLYCOL | B | 0,5-4 | 0,5-4 | 0,5-4 | 2 |
| GLYCERYN | B | - | - | - | 4 |
| 1,2-HEXANEDIOL | B | 0,01-1 | 0,01-1 | 0,01-1 | 0,3 |
| CAPRYLYL GLYCOL | B | 0,01-1 | 0,01-1 | 0,01-1 | 0,3 |
| HYDROXYACETOPHENONE | B | 0,1-0,7 | 0,1-0,7 | 0,1-0,7 | 0,4 |
| XANTHAN GUM | C | 0,1-1 | 0,1-1 | 0,1-1 | 0,5 |
| SODIUM STEAROYL GLUTAMATE | D | 0,5-2 | 0,5-2 | 0,5-2 | - |
| STEARYL ALCOHOL | E | 0,5-4 | 0,5-4 | 0,5-4 | 2 |
| POLYGLYCERYL-2-DIPOLYHYDROXYSTEARATE | E | - | - | - | 4 |
| DISODIUM CETEARYL SULFOSUCCINATE | e | - | - | - | 1,5 |
| VP/EICOSENE COPOLYMER | E | 2,5 | - | - | - |
| POLYGLYCERYL-3 DICITRATE/STEARATE | E | 1,5-4 | 1,5-4 | 1,5-4 | - |
| DIISOSTEAROYL POLYGLYCERYL-3 DIMER DILINOLEATE | E | - | 1,5-2,5 | 1,5-2,5 | 2,4 |
| CAPRYLIC / CAPRIC TRIGLYCERIDE | E | - | 1-3 | 1-3 | 1,6 |
| DIBUTYL ADIPATE | E | 2-10 | 2-10 | 2-10 | 3 |
| C12-15 ALKYL BENZOATE | E | 2-10 | 2-10 | 2-10 | 2 |
| DICAPRYLYL CARBONATE | E | 2-10 | 2-10 | 2-10 | 8 |
| PHENOXYETHYL CAPRYLATE | E | 2-10 | 2-10 | 2-10 | 10 |
| DIETHYLAMINO HYDROXYBENZOYL HEXYL BENZOATE | E | 5-10 | 5-10 | 5-10 | 6 |
| BIS-ETHYLHEXYLOXYPHENOL METHOXYPHENYL TRIAZINE | E | 2-8 | 2-8 | 2-8 | 3 |
| ETHYLHEXYL TRIAZONE | E | 1-5 | 1-5 | 1-5 | 4 |
| AQUA | F | - | - | - | |
| POLYURETHANE-35 | F | - | - | - | - |
| AQUA | F | - | - | 0,75-2,25 | - |
| POLYURETHANE-34 | F | - | - | 0,5-1,5 | - |
| AQUA | F | - | - | - | 3 |
| POLYURETHAN E-99 | F | - | - | - | 2 |
| AQUA | G | 1-2 | 1-2 | 1-2 | - |
| CITRIC ACID | G | 0,1-0,2 | 0,1-0,2 | 0,1-0,2 | - |
| TOTAL | - | 100 | 100 | 100 | 100 |

**[Table 2]**

| INCI | PHASE | LH3 | LH4 | H4 (hors invention) |
|---|---|---|---|---|
| AQUA | A | 45-65 | 45-65 | 45-65 |
| POTASSIUM GLUCONATE | A | 0,01-0,5 | 0,01-0,5 | 0,01-0,5 |
| PENTYLENE GLYCOL | B | 0,5-4 | 0,5-4 | 0,5-4 |
| 1,2-HEXANEDIOL | B | 0,01-1 | 0,01-1 | 0,01-1 |
| CAPRYLYL GLYCOL | B | 0,01-1 | 0,01-1 | 0,01-1 |
| HYDROXYACETOPHENONE | B | 0,1-0,7 | 0,1-0,7 | 0,1-0,7 |
| XANTHAN GUM | C | 0,1-1 | 0,1-1 | 0,1-1 |
| SODIUM STEAROYL GLUTAMATE | D | 0,5-2 | 0,5-2 | 0,5-2 |
| STEARYL ALCOHOL | E | 0,5-4 | 0,5-4 | 0,5-4 |
| VP/EICOSENE COPOLYMER | E | - | - | - |
| POLYGLYCERYL-3 DICITRATE/STEARATE | E | 1,5-4 | 1,5-4 | 1,5-4 |
| DIISOSTEAROYL POLYGLYCERYL-3 DIMER DILINOLEATE | E | 1,5-2,5 | 1,5-2,5 | - |
| CAPRYLIC / CAPRIC TRIGLYCERIDE | E | 1-3 | 1-3 | - |
| DIBUTYL ADIPATE | E | 2-10 | 2-10 | 2-10 |
| C12-15 ALKYL BENZOATE | E | 2-10 | 2-10 | 2-10 |
| DICAPRYLYL CARBONATE | E | 2-10 | 2-10 | 2-10 |
| PHENOXYETHYL CAPRYLATE | E | 2-10 | 2-10 | 2-10 |
| DIETHYLAMINO HYDROXYBENZOYL HEXYL BENZOATE | E | 5-10 | 5-10 | 5-10 |
| BIS-ETHYLHEXYLOXYPHENOL METHOXYPHENYL TRIAZINE | E | 2-8 | 2-8 | 2-8 |
| ETHYLHEXYL TRIAZONE | E | 1-5 | 1-5 | 1-5 |
| AQUA | F | - | 1,5-7 | 1,5-7 |
| POLYURETHANE-35 | F | - | 1-4,5 | 1-4,5 |
| AQUA | F | - | - | - |
| POLYURETHAN E-34 | F | - | - | - |
| AQUA | F | 1,5-7 | - | - |
| POLYURETHAN E-99 | F | 1-4,5 | - | - |
| AQUA | G | 1-2 | 1-2 | 1-2 |
| CITRIC ACID | G | 0,1-0,2 | 0,1-0,2 | 0,1-0,2 |
| TOTAL | - | 100 | 100 | 100 |

Tableaux 1 et 2 : Compositions massiques (en %) des compositions de référence (hors invention), L1 (hors invention), LH1, LH2, LH3, LH4 et H4 (hors invention).

### Procédé de préparation des sept compositions :

La phase aqueuse (A) est homogénéisée sous défloculeuse Raynerie. La phase de glycol (B) est homogénéisée à 70°C. Le gélifiant (C) est dispersé dans B sous agitation magnétique. La phase B+C est ajoutée à la phase A sous forte agitation pour déployer le gel. La phase aqueuse gélifiée (ABC) est chauffée sous agitation jusqu'à une 85°C. Lorsque la phase ABC atteint 65°C, la phase D (si présente) y est ajoutée en maintenant l'agitation. La phase grasse (E) est préparée en parallèle par le mélange de l'ensemble de ses composants et une homogénéisation sous agitation magnétique sous une chauffe à 85°C. Lorsque la phase aqueuse (ABCD ou ABC) est homogène, à 85°C et la phase grasse (E) est également à 85°C l'émulsion est mise en oeuvre par l'incorporation de la phase grasse (E) dans la phase aqueuse (ABCD ou ABC) sous forte agitation et la chauffe est coupée .La composition est ensuite refroidie sous agitation lente jusqu'à la température ambiante (environ 20-25°C).Lorsqu'elle est présente la phase de filmogène hydrophile (F) est incorporée dès que la composition atteint une température inférieure à 50°C.Le ph est ajusté à froid par l'addition d'une phase de solution acide (G) afin d'obtenir un pH compris entre 7,5 et 8 (cas LH2) ou entre 5 et 5,5 (autres cas).

### Exemple 2 : efficacité de protection solaire des compositions de l'exemple 1

L'étude de l'efficacité de protection solaire (SPF) a été démontrée via la méthode de mesure décrite par la norme ISO 24444 :2020. Les résultats sont compilés dans le tableau 3.

### Exemple 3 : résistance à l'eau des compositions de l'exemple 1

L'étude de l'efficacité de protection solaire (SPF) a été démontrée via la méthode de mesure décrite par la norme ISO 24444 :2020.

L'étude de l'efficacité de résistance à l'eau de la protection solaire a été démontrée via la méthode de mesure décrite par la norme ISO 18861 :2020. Les résultats sont compilés dans le tableau 3.

**[Table 3]**

| Efficacité et résistance | Réf. (hors invention) | L1 (hors invention) | LH1 | LH3 | H4 (hors invention) | LH4 |
|---|---|---|---|---|---|---|
| Nature Filmogène | lipophile | lipophile | lipophile + hydrophile | lipophile + hydrophile | hydrophile | lipophile + hydrophile |
| % matière filmogène | 2,5 | 1,5 | 2,3 | 3,5 | 2 | 3,5 |
| % gain SPF par rapport à Réf. | 0 | -8.6 | +6,9 | +9,1 | +0,9 | +19,7 |
| % gain Résistance à l'eau par rapport à Réf. | 0 | +7,0 | +16,9 | +17,9 | +4,5 | +18,9 |

Tableau 3 : Efficacité de protection solaire et résistance à l'eau des compositions référence (hors invention) et L1 (hors invention), LH1, H3 (hors invention), H4, et H3 (hors invention) par rapport à la composition référence.

### Liste de références

**(1)** Couteau C, Coiffard L. Produit de protection solaire - Formulation et efficacité. Techniquesde l'ingénieur (2018).
**(2)** Hubaud J-C. Formulation et évaluation de produits solaire - Evaluation. Conception des produits cosmétiques (2015).
**(3)** Jansen R, Wang S, et al. Photoprotection - Part I. Photoprotection by naturally occurring, physical and systemic agents. J Am Acad Dermatol (2013), 69: 853.
**(4)** http://www.planetoscope.com/eau-oceans/1247-litres-de-creme-solaire-diluee-dans-locean.html.
**(5)** https://www.nytimes.com/2018/05/03/travel/hawaii-sunscreen-ban.html.
**(6)** Jovanovic B, Guzman H. Effects of titanium oxy (TiC2) nanoparticles on caribean reef building coral (monstastraea faveolata). Environ Toxicol Chem (2014), 33: 1346.
**(7)** Downs C, Karmarsky-Winter A, et al. Toxicophatological effects of the sunscreen UV filter, oxybenzone (benzophenone-3), on coral planulae and cultured primary cells and its environmental contamination in Hawaii and the U.S Virgin Islands. Arch Environ Contam Toxicol (2016), 70: 265.
**(8)** Danovaro R, Bongiorni L, et al. Sunscreens cause coral bleaching by promoting viral infections. Environmental Health Perspectives (2008).
**(9)** Sanchez-Quiles D, Tovar-Sanchez A. Are sunscreens a new environemental riskeassociated with coastal tourism? Environment International (2015) 83: 158.
**(10)** https://www.nytimes.com/2018/05/03/travel/hawaii-sunscreen-ban.html.
**(11)** http://www.premiumbeautynews.com/fr/la-france-va-inventorier-les, 13745.

## Revendications

1. Composition cosmétique comprenant au moins un filtre UVA et/ou UVB, un agent filmogène lipophile comprenant du diisostearoyl polyglyceryl 3 dimer dilinoleate et un agent filmogène hydrophile comprenant un polyuréthane.

2. Composition selon la revendication 1, dans laquelle la concentration massique en diisostearoyl polyglyceryl 3 dimer dilinoleate est comprise dans un intervalle allant de 0,6 à 6 %, de préférence 1,5 % à 2,25 %, en masse par rapport à la masse totale de la composition.

3. Composition selon l'une quelconque des revendications précédentes, dans laquelle la concentration massique en polyuréthane est comprise dans un intervalle allant de 0,5 à 5 %, de préférence 1 à 4,5 %, en masse par rapport à la masse totale de la composition.

4. Composition selon l'une quelconque des revendications précédentes, dans laquelle le filtre UVA et/ou UVB est choisi parmi l'homosalate, l'éthylhéxyl salicylate, le bis-éthylhéxyloxyphénol méthoxyphényl triazine, l'octocrylène, le butyl méthoxydibenzoylméthane, l'éthylhéxyl triazone, le Diethylamino Hydroxybenzoyl Hexyl Benzoate, le diéthylhexyl butamido triazone, le Methylene Bis-Benzotriazolyl Tetramethylbutylphenol, le Phenylbenzimidazol Sulfonic Acid, le Disodium Phenyl Dibenzimidazole Tetrasulfonate, Ethylhexyl Methoxycinnamate, Isoamyl p-Methoxycinnamate et leurs mélanges, de préférence parmi le le bis-éthylhéxyloxyphénol méthoxyphényl triazine, le butyl méthoxydibenzoylméthane, l'éthylhéxyl triazone, le Diethylamino Hydroxybenzoyl Hexyl Benzoate, le diéthylhexyl butamido triazone et leurs mélanges, et de manière encore préférée parmi le le bis-éthylhéxyloxyphénol méthoxyphényl triazine, l'éthylhéxyl triazone, le Diethylamino Hydroxybenzoyl Hexyl Benzoate , et leurs mélanges.

5. Composition selon l'une quelconque des revendications précédentes, dans laquelle le polyuréthane est choisi parmi le polyuréthane-34, le polyuréthane-35, le polyuréthane-48, le polyuréthane-93, le polyuréthane-99 et leurs mélanges, de préférence parmi le polyuréthane-35, le polyuréthane-99 et leurs mélanges.

6. Composition selon l'une quelconque des revendications précédentes, dans laquelle la concentration massique en filtre UVA et/ou UVB peut être comprise dans un intervalle allant de 1 à 50 %, de préférence de 5 à 20 % en masse par rapport à la masse totale de la composition.

7. Composition selon l'une quelconque des revendications précédentes comprenant en outre un ou plusieurs excipients cosmétiques, de préférence choisis parmi les excipients cosmétiques hydrophiles et/ou lipophiles, et de manière encore préférée choisi parmi :
- un parfum, un colorant, un pigment, une huile, de préférence d'origine végétale, un ester, un carbonate, un alcane, un ether, un alcool gras, un acide gras, un antioxydant, une huile essentielle et un actif cosmétique lipophile, et/ou
- un tensioactif, un colorant, un polyol, un gélifiant (i.e. un polysaccharide, dont notamment les gommes et/ou un carbomer), un agent régulateur de pH, un conservateur, un agent antimicrobien, un agent chélatant et un actif cosmétique hydrophile.

8. Composition selon l'une quelconque des revendications précédentes, ladite composition étant sous forme :
- d'une émulsion huile-dans-eau, de préférence une crème, un lait, un spray, une émulsion coulée et/ou d'une émulsion colorée telle qu'une BB crème ou un fond de teint),
- d'une émulsion eau-dans-huile, de préférence une crème, un lait, un spray, une émulsion coulée et/ou d'une émulsion colorée telle qu'une BB crème ou fond de teint, ou
- d'une composition bi-phasique.

9. Utilisation d'une combinaison d'un agent filmogène lipophile comprenant du diisostearoyl polyglyceryl 3 dimer dilinoleate et d'un agent filmogène hydrophile comprenant un polyuréthane dans une composition cosmétique de protection solaire.

10. Utilisation selon la revendication précédente dans laquelle la composition cosmétique de protection solaire se présente sous la forme :
- d'une émulsion huile-dans-eau, de préférence une crème, un lait, un spray, une émulsion coulée et/ou d'une émulsion colorée telle qu'une BB crème ou un fond de teint),
- d'une émulsion eau-dans-huile, de préférence une crème, un lait, un spray, une émulsion coulée et/ou d'une émulsion colorée telle qu'une BB crème ou fond de teint, ou
- d'une composition bi-phasique.
